# EUROPÄISCHE PATENTANMELDUNG

(11) **EP 1 502 644 A2**
(43) Veröffentlichungstag der Anmeldung: **02.02.2005**
(21) Anmeldenummer: 04016922.9
(22) Anmeldetag: 17.07.2004
(51) Int. Cl.: B01F 17/00, A61K 7/50

(54) **Emulgatorkombination, diese enthaltende Emulsion und Verfahren zu deren Herstellung**

(30) Priorität: 28.07.2003 DE 10334225; 02.10.2003 DE 10346515
(71) Anmelder: Cognis Deutschland GmbH & Co. KG, 40589 Düsseldorf (DE)
(72) Erfinder: Issberner, Ulrich, Dr., 41569 Rommerkirchen (DE); Goget, Caroline, 40213 Düsseldorf (DE)

(57) **Zusammenfassung**

Die Erfindung betrifft eine Emulgatorkombination, welche frei ist von ethoxylierten Emulgatoren, weniger als 20 Gew.-% Wasser enthält und C₈-C₁₄-Alkyloligoglucosid, Polyolpolyhydroxystearat und Acylglutamat umfasst. Die Emulgatorkombination eignet sich zur Herstellung von Nanoemulsionen in herkömmlichen Kalt/Kalt- oder Heiß/Heiß-Verfahren. Die Nanoemulsionen sowie ein Verfahren zu deren Herstellung sowie Tücher, die mit den Nanoemulsionen beschichtet sind, sind ebenfalls Gegenstand der Erfindung.

## Beschreibung

### Gebiet der Erfindung

Gegenstand der Erfindung ist eine Emulgatorkombination, die, obgleich sie frei ist von ethoxylierten Tensiden, in herkömmlichen Kalt/Kalt- oder Heiß/Heiß-Emulgationsverfahren stabile Nanoemulsionen liefert. Die unter Verwendung der erfindungsgemäßen Emulgatorkombination hergestellten Nanoemulsionen sowie ein Verfahren zu deren Herstellung sind ebenfalls Gegenstand der Erfindung.

### Stand der Technik

Unter Nanoemulsionen werden üblicherweise Emulsionen verstanden, deren Teilchen- bzw. Tröpfchengröße bei unter 1000 nm liegt. Insbesondere versteht man unter Nanoemulsionen solche mit einer durchschnittlichen Teilchengröße von etwa 5 bis 500 nm.
Nanoemulsionen finden wegen ihrer günstigen Eigenschaften häufig Anwendung in kosmetischen und pharmazeutischen Zubereitungen. Vorteilhaft sind vor allem die Phasenstabilität auch bei geringen Viskositäten sowie die gegenüber herkömmlichen Emulsionen deutlich erhöhte Resorptionsrate für aktive Wirkstoffe, die mit der Emulsion zum Beispiel auf Haut oder Haar aufgebracht werden.

Stabile Nanoemulsionen werden bisher fast ausschließlich nach der Phaseninversionsmethode (PIT-Verfahren) erhalten. Hierbei können jedoch nur ethoxylierte Emulgatoren eingesetzt werden. Diese sind jedoch oft hautreizend und von daher nachteilig. Nach dem PIT-Verfahren erhaltene Emulsionen sind beispielsweise in der DE 19541754 A1 der Anmelderin beschrieben.

Ebenfalls von der Anmelderin wurde in der DE 10059430 A1 ein alternatives Verfahren zum PIT-Prozess aufgezeigt, in dem die ethoxylierten Emulgatoren zumindest teilweise durch nichtethoxylierte ersetzt werden können. Die Emulgierung von wässriger Phase und Ölphase erfolgt hier durch Hochdruckhomogenisierung. Das Herstellungsverfahren ist damit relativ aufwändig und teuer.

Es bestand daher ein Bedarf an einem Emulgator bzw. einer Emulgatorkombination, in der keine ethoxylierten Emulgatoren eingesetzt werden müssen, um dennoch auf einfache und kostengünstige Weise stabile Nanoemulsionen zu erhalten. Aufgabe der Erfindung ist es entsprechend, eine derartige Emulgatorkombination zur Verfügung zu stellen. Eine weiterer Aspekt der Aufgabe war es, Produkte bereitzustellen, die mit ethoxylat-freien Nanoemulsionen beschichtet sind.

### Beschreibung der Erfindung

Die Lösung der Aufgabe gelingt mit der Emulgatorkombination nach Anspruch 1. Bevorzugte Ausführungsformen sind in den Unteransprüchen beschrieben. In einem ersten Aspekt betrifft die Erfindung daher eine Emulgatorkombination, welche frei ist von ethoxylierten nichtionischen Emulgatoren, weniger als 20 Gew.-% Wasser enthält und welche a) C₈-C₁₄-Alkyloligoglykosid, b) Polyolpolyhydroxystearat und c) C₆-C₂₂-Acylglutamat umfasst.

Unter der Bezeichnung Emulgator sollen hier sowohl Emulgatoren als auch Tenside verstanden werden, also oberflächenaktive Stoffe insgesamt. Im Folgenden wird stellvertretend für alle derartigen Verbindungen der Begriff "Emulgator" verwendet.

### Alkyloligioglykosid

Alkyloligoglykoside stellen bekannte nichtionische Tenside dar, die der Formel (I) folgen,

**R**^{**1**}**O-[G]**_{**p**} **(I)**

in der R¹ für einen Alkylrest, G für einen Zuckerrest mit 5 oder 6 Kohlenstoffatomen und p für Zahlen von 1 bis 10 steht. Sie können nach den einschlägigen Verfahren der präparativen organischen Chemie erhalten werden. Stellvertretend für das umfangreiche Schrifttum sei hier auf die Schriften **EP 0301298 A1** und **WO 90/03977 A** verwiesen.

Die Alkyloligoglykoside können sich von Aldosen bzw. Ketosen mit 5 oder 6 Kohlenstoffatomen, vorzugsweise der Glucose, ableiten. Die bevorzugten Alkyloligoglykoside sind somit Alkyloligoglucoside.

Die Indexzahl p in der allgemeinen Formel (I) gibt den Oligomerisierungsgrad (DP-Grad), d. h. die Verteilung von Mono- und Oligoglykosiden, an und steht für eine Zahl zwischen 1 und 10. Während p in einer gegebenen Verbindung stets ganzzahlig sein muss und hier vor allem die Werte p = 1 bis 6 annehmen kann, ist der Wert p für ein bestimmtes Alkyloligoglykosid eine analytisch ermittelte rechnerische Größe, die meistens eine gebrochene Zahl darstellt. Vorzugsweise werden Alkyloligoglykoside mit einem mittleren Oligomerisierungsgrad p von 1,1 bis 3,0 eingesetzt. Aus anwendungstechnischer Sicht sind solche Alkyloligoglykoside bevorzugt, deren Oligomerisierungsgrad kleiner als 1,7 ist und insbesondere zwischen 1,2 und 1,4 liegt.

Im Rahmen der Erfindung wurde festgestellt, dass der Länge des Alkylrestes R¹ offenbar eine besondere Bedeutung dabei zukommt, dass sich die erfindungsgemäße Emulgatorkombination zur Herstellung von Nanoemulsionen eignet. Erfindungsgemäß werden daher Alkyloligoglykoside verwendet, in denen sich der Rest R¹ von primären Alkoholen mit 8 bis 14 Kohlenstoffatomen ableitet. Es können auch technische Gemische der Alkohole eingesetzt werden. Bevorzugt sind die C₈- bis C₁₂-Alkylreste. Wie erwähnt, sind die entsprechenden Alkyloligoglucoside besonders geeignet, insbesondere wird Laurylglucosid bevorzugt eingesetzt.

### Polyolpolyhydroxystearat

Polyolpolyhydroxystearate sind Ester von Polyolen und Polyhydroxystearinsäuren. Die Polyolkomponente kann sich beispielsweise von Glycerin, Ethylenglycol, Diethylenglycol, Propylenglycol, Polyglycerin, Trimethylolethan, Trimethylolpropan, Trimethylolbutan, Pentaerythrit, Dipentaerythrit, Methyl- und Butylglucosid, Sorbit, Mannit, Glucose, Saccharose oder Glucamin ableiten. Entsprechende Stoffe sind beispielsweise aus den Druckschriften **GB-A-1524782** oder **EP-A-0000424** bekannt.

Vorzugsweise handelt es sich bei den Stoffen der Komponente b) um Polyglycerinpolyhydroxystearate, die man erhält, indem man Polyhydroxystearinsäure mit einem Eigenkondensationsgrad im Bereich von 2 bis 20, vorzugsweise 2 bis 10, mit einem Polyglyceringemisch der bevorzugten Zusammensetzung (GC-Methode)

| | |
|---|---|
| Glycerin | 5 bis 35 (15 bis 30) Gew.-% |
| Diglycerine | 15 bis 40 (20 bis 32) Gew.-% |
| Triglycerine | 10 bis 35 (15 bis 25) Gew.-% |
| Tetraglycerine | 5 bis 20 ( 8 bis 15) Gew.-% |
| Pentaglycerine | 2 bis 10 ( 3 bis 8) Gew.-% |
| Oligoglycerine | ad 100 Gew.-% |

in an sich bekannter Weise verestert; in Klammern angegeben sind die bevorzugten Bereiche. Die Herstellung der Polyolpolyhydroxystearate kann in an sich bekannter Weise erfolgen. Im Fall der Polyglycerinpolyhydroxystearate wird dabei vorzugsweise zunächst das Polyglycerin und dann die Polyhydroxystearinsäure hergestellt, und schließlich werden beide verestert. Die Herstellung eines Polyglycerins der oben genannten Zusammensetzung kann durch Eigenkondensation von Glycerin in Gegenwart von geeigneten Katalysatoren wie beispielsweise Kaliumcarbonat, Silicaten gemäß **DE 4029323 A1** (Henkel) oder Boraten gemäß **DE 4117033 A1** (Henkel) bei Temperaturen im Bereich von 200 bis 260 °C durchgeführt werden. Die Herstellung der Polyhydroxystearinsäure erfolgt beispielsweise durch alkalisch katalysierte Polykondensation von Hydroxystearinsäure, vorzugsweise 12-Hydroxystearinsäure, die durch Härtung von Ricinolsäure bzw. technischer Ricinusölfettsäure gewonnen wird. Vorzugsweise werden dabei lineare Veresterungsprodukte mit 2 bis 10 und insbesondere 2 bis 8 Fettsäureeinheiten gebildet. Typischerweise wird die folgende Verteilung (GPC-Methode) erreicht:

| | |
|---|---|
| Monomere | 1 bis 10 Gew.-% |
| Dimere | 5 bis 15 Gew.-% |
| Trimere | 5 bis 15 Gew.-% |
| Tetramere | 5 bis 15 Gew.-% |
| Pentamere | 5 bis 15 Gew.-% |
| Hexamere | 5 bis 15 Gew.-% |
| Heptamere | 5 bis 15 Gew.-% |
| Octamere | 1 bis 10 Gew.-% |
| Oligomere | ad 100 Gew.-% |

In einer besonderen Ausführungsform der Erfindung werden Gemische von Hydroxystearinsäure und Ricinolsäure bzw. technischer Ricinusölfettsäure, die zu etwa 90 Gew.-% aus Ricinolsäure besteht, im Gewichtsverhältnis 99 : 1 bis 1 : 99 und vorzugsweise 75 : 25 bis 10 : 90 eingesetzt. In gleicher Weise ist es möglich, die Säuren einzeln zu kondensieren und anschließend die Kondensate abzumischen. Bei der nachfolgenden Kondensation der Polyolkomponente, beispielsweise des Polyglycerins mit der Polyhydroxystearinsäure bzw. den Gemischen mit Polyricinolsäure, wird eine komplexe Mischung homologer Polyester gebildet.. Kondensationsprodukte auf Basis von Polyglycerin und Polyhydroxystearinsäure bzw. Polyhydroxystearinsäure/Polyricinolsäure können über ihre Jodzahl charakterisiert werden. Typische Beispiele sind Polyester mit einer lodzahl < 10 (Basis 100% 12-Hydroxystearinsäure) bzw. 65 bis 80 (Basis 90 % 12-Hydroxystearinsäure, 10 % Ricinolsäure).

### Acylglutamat

Acylglutamate sind bekannte anionische Tenside dar, die der Formel (II) folgen,

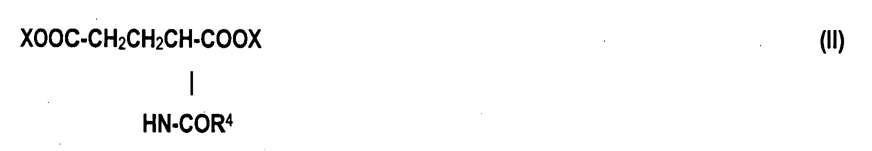

in der R⁴CO für einen linearen oder verzweigten Acylrest mit 6 bis 22 Kohlenstoffatomen und 0, 1, 2 oder 3 Doppelbindungen und X für Wasserstoff, ein Alkali- und/oder Erdalkalimetall, Ammonium, Alkylammonium, Alkanolammonium oder Glucammonium steht. Ihre Herstellung erfolgt beispielsweise durch Schotten-Baumann-Acylierung von Glutaminsäure mit Fettsäuren, Fettsäureestern oder - chloriden. Verkaufsprodukte sind beispielsweise von der Hoechst AG, Frankfurt/DE oder der Ajinomoto Co. Inc., Tokio/JP erhältlich. Eine Übersicht zu Herstellung und Eigenschaften der Acylglutamate findet sich von M. Takehara et al. in **J. Am. Oil Chem. Soc. 49 (1972) 143.** Typische Beispiele für geeignete Acylglutamate, die im Sinne der Erfindung in Betracht kommen, sind diejenigen Acylglutamate, die sich von Fettsäuren mit 6 bis 22 und vorzugsweise 12 bis 18 Kohlenstoffatomen ableiten. Besonders bevorzugt sind Kokosfettsäureglutamate, beispielsweise der C_{12/14}- bzw. C_{12/18}-Kokosfettsäure. Es werden insbesondere die Mono- oder Dialkalimetallsalze des Acylglutamats eingesetzt.

Die Emulgatorkonzentrate eignen sich auch, um Parfümöle zu solubilisieren. Ein weiterer Gegenstand der Anmeldung ist daher die Verwendung der erfindungsgemäßen Emulgatorkonzentrate zur Solubilisierung von Parfümölen.

### Weitere Emulgatoren

Zusätzlich zu den genannten Komponenten a) bis c) kann die erfindungsgemäße Emulgatorkombination weitere mit den Komponenten a) bis c) kompatible Emulgatoren oder Tenside enthalten. Die Auswahl richtet sich zweckmäßig nach der beabsichtigten Verwendung der Emulgatorkombination und insbesondere nach der Art des Ölkörpers, der emulgiert werden soll. Ethoxylierte Emulgatoren zählen erfindungsgemäß nicht zu den weiteren Emulgatoren. Als zusätzliche Komponente ist bevorzugt mindestens ein weiteres nichtionisches Tensid enthalten. Besonders geeignet sind Sorbitanmono- oder -diester einer gesättigten oder ungesättigten Fettsäure mit 6 bis 22 und bevorzugt 12 bis 20 Kohlenstoffatomen, insbesondere Sorbitanstearat.

Auch die Mengen, in denen die Komponenten a) bis c) und gegebenenfalls die weiteren Emulgatoren zueinander in der erfindungsgemäßen Emulgatorzusammensetzung verwendet werden, richten sich nach der Zusammensetzung der Ölphase, die emulgiert werden soll. Zweckmäßig hat sich ein Anteil der Komponente c) in der Emulgatorkombination von 1 bis 50 Gew.-%, bevorzugt 3 bis 15 Gew.-% und insbesondere 5 bis 11 Gew.-% erwiesen.

Das Gewichtsverhältnis der beiden Komponenten a) und b) liegt zweckmäßig bei 5 : 1 bis 1 : 5 und bevorzugt 2 : 1 bis 1 : 2. Besonders günstig ist es, wenn beide Komponenten im Wesentlichen in gleichen Gewichtsanteilen enthalten sind.

Wird in der erfindungsgemäßen Emulgatorkombination ein weiteres Tensid/ein weiterer Emulgator eingesetzt liegt sein Gewichtsanteil zur Gesamtmenge der Komponenten a) und b) zweckmäßig bei 1 : 1 bis 1 : 10 und bevorzugt bei 1 : 2 bis 1 : 5.

Die erfindungsgemäße Emulgatorkombination kann in Form eines Konzentrats sämtlicher der genannten Emulgatorkomponenten mit einem Anteil von weniger als 20 Gew.-% Wasser zum Einsatz kommen. Der Wasseranteil der erfindungsgemäßen Emulgatorkombination liegt bevorzugt bei unter 10 Gew.-% und besonders bevorzugt bei weniger als 5 Gew.-%.

Von Vorteil wird der Emulgatorkombination Glycerin zugesetzt, welches in Kombination mit dem wässrigen Anteil zu einer flüssigen und pumpfähigen Emulgatorzusammensetzung führt.

Es ist jedoch auch möglich, die erfindungsgemäße Emulgatorkombination in Form ihrer Einzelkomponenten oder in Form von Mischungen der Einzelkomponenten einzusetzen, wobei dann der Wasseranteil der Gesamtheit der Einzelkomponenten oder deren Mischungen unter 20 Gew.-% liegt. Bei der Herstellung einer Emulsion kann die erfindungsgemäße Emulgatorkombination also nicht nur insgesamt mit der Ölphase vorgelegt werden, sondern es ist ebenfalls möglich, die Komponenten der Emulgatorkombination auf die Ölphase und die wässrige Phase aufzuteilen und zwar zweckmäßig derart, dass die öllöslichen Komponenten der Ölphase zugesetzt werden und die wasserlöslichen der wässrigen Phase.

### Nanoemulsion

Die erfindungsgemäße Emulgatorkombination eignet sich grundsätzlich zur Herstellung von Nanoemulsionen aus allen üblicherweise in kosmetischen oder pharmazeutischen Emulsionen verwendeten Ölkörpern/Emollients. Ein weiterer Gegenstand der Erfindung sind daher Nanoemulsionen, in der eine Ölphase und eine wässrige Phase miteinander emulgiert sind und in welcher als Emulgator die beschriebene Emulgatorkombination vorhanden ist und die frei ist von ethoxylierten Emulgatoren. Erfindungsgemäß sollen unter dem Begriff Nanoemulsion Emulsionen mit einer Teilchen- bzw. Tröpfchengröße von unter 1000 nm verstanden werden. Üblicherweise liegt die Teilchengröße der erfindungsgemäßen Nanoemulsion im Bereich von 5 bis 500 nm und insbesondere bei 50 bis 200 nm.

Als Ölkörper kommen beispielsweise Guerbetalkohole auf Basis von Fettalkoholen mit 6 bis 18, vorzugsweise 8 bis 10 Kohlenstoffatomen (z. B. Eutanol® G) , Ester von linearen C₆-C₂₂-Fettsäuren mit linearen oder verzweigten C₆-C₂₂-Fettalkoholen bzw. Ester von verzweigten C₆-C₁₃-Carbonsäuren mit linearen oder verzweigten C₆-C₂₂-Fettalkoholen, wie z.B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Daneben eignen sich Ester von linearen C₆-C₂₂-Fettsäuren mit verzweigten Alkoholen, insbesondere 2-Ethylhexanol, Ester von C₃-C₃₈-Alkylhydroxycarbonsäuren mit linearen oder verzweigten C₆-C₂₂-Fettalkoholen - insbesondere Dioctylmalat -, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane wie z.B. 1,3-Dialkylcyclohexane, lineare und verzweigte C₆-C₂₂-Fettalkoholcarbonate, wie z.B. Dicaprylyl Carbonate (Cetiol® CC), Guerbetcarbonate auf Basis von Fettalkoholen mit 6 bis 18, vorzugsweise 8 bis 10 C Atomen, Ester der Benzoesäure mit linearen und/oder verzweigten C₆-C₂₂-Alkoholen (z.B. Finsolv® TN), lineare oder verzweigte, symmetrische oder unsymmetrische Dialkylether mit 6 bis 22 Kohlenstoffatomen pro Alkylgruppe, wie z.B. Dicaprylyl Ether (Cetiol® OE), Ringöffnungsprodukte von epoxidierten Fettsäureestern mit Polyolen (Hydagen® HSP, Sovermol® 750, Sovermol® 1102), Siliconöle (Cyclomethicone, Siliciummethicontypen u.a.) und/oder aliphatische bzw. naphthenische Kohlenwasserstoffe, wie z.B. wie Mineralöl, Vaseline, Petrolatum, Squalan, Squalen oder Dialkylcyclohexane in Betracht.

In der erfindungsgemäßen Nanoemulsion bevorzugte Ölkörper sind ausgewählt aus Fettsäureestern von C₆-C₂₂-Fettsäuren mit C₆-C₂₂-Fettalkoholen, C₆-C₂₂-Fettalkoholcarbonaten, Siliconölen, 1,3-Dialkylcyclohexanen und C₆-C₂₂-Dialkylethern.

Besonders gute Ergebnisse werden erzielt, wenn die Nanoemulsion folgende Komponenten umfasst:
a) C₈-C₁₂-Alkyloligoglucosid, insbesondere Laurylglucosid,
b) Polyglycerinpolyhydroxystearat,
c) C₁₂-C₁₈-Acylglutamat., insbesondere Kokosfettsäureglutamat,
d) Sorbitanester einer C₁₆-C₂₀-Fettsäure, insbesondere Sorbitanstearat, wenigstens einen Ölkörper und bevorzugt wenigstens einen Ölkörper pro Gruppe, ausgewählt aus:
e) Fettsäureestern von C₆-C₂₀-Fettsäuren mit C₆-C₂₀-Fettalkoholen, insbesondere Hexyllaurat, Ethylhexylpalmitat und Cetearylisononanoat,
f) C₆-C₁₂-Dialkylcarbonaten, insbesondere Dicaprylylcarbonat, und
g) Cyclomethiconen und Siliciummethiconen, insbesondere Dimethiconen, sowie
h) Glycerin,
i) Wasser und gegebenenfalls
j) aktiven Wirkstoffe, Hilfs- und Zusatzstoffe.

Anstelle der Einzelkomponenten a), b) und i) kann vorteilhaft auch eine Emulgatorzusammensetzung der Cognis Deutschland GmbH & Co. KG verwendet werden, die unter dem Handelsnamen Eumulgin® VL 75 erhältlich ist und Laurylglucosid, Polyglyceryl-2-dipolyhydroxystearat und Glycerin enthält.

Vorzugsweise enthält die Nanoemulsion zusätzlich C₁₆-C₂₂-Fettalkohol(e), insbesondere Cetyl- und Cetylstearylalkohol, in einer Menge von vorzugsweise 1,0 -10 Gew.-%.

Unter **aktiven Wirkstoffen** sind dabei unter anderem Arzneimittel, kosmetische oder pharmazeutische Wirkstoffe wie beispielsweise Antioxidantien, UV-Filter, biogene Stoffe, Deodorantien, Selbstbräuner, Depigmentierungsmittel, Parfümöle oder sonstige Duftstoffe usw. zu verstehen. Auch Hydrotrope, Füllstoffe, Quellmittel, Stabilisatoren (zum Beispiel Polysaccharid-, Acrylsäure-, Acrylamid-Polymere), Konservierungsmittel, Farbstoffe und die übrigen gängigen Hilfs- und Zusatzstoffe, die üblicherweise nur in geringen Mengen eingesetzt werden, können vorhanden sein.

Die Menge, in der die erfindungsgemäße Emulgatorkombination zum Emulgieren eingesetzt wird, richtet sich in grundsätzlich bekannter Weise nach Art und Zusammensetzung des zu emulgierenden Ölkörpers. Vorzugsweise liegt der Anteil an Komponente c) bei 0,05 bis 3 Gew.-% und insbesondere bei 0,1 bis 1,5 Gew.-%, bezogen auf die Gesamtmenge der Nanoemulsion. Der Anteil an zusätzlichen Emulgatorkomponenten beträgt in der Regel 0,1 bis 15 Gew.-% und insbesondere 1 bis 10 Gew.-%, ebenfalls bezogen auf die Gesamtmenge der Nanoemulsion. Ölkörper/Emollient ist in der Nanoemulsion zweckmäßig in einer Menge von 1 bis 50 Gew.-% und insbesondere 1 bis 30 Gew.-%, bezogen auf die Gesamtmenge der Nanoemulsion, vorhanden.

Der Wasseranteil liegt im Allgemeinen bei über 20 Gew.-%, bevorzugt bei über 40 Gew.-% und insbesondere bei über 50 Gew.-%.

Eine ganz besonders bevorzugte Ausführungsform der Nanoemulsion umfasst:
a) 0,5 -5,0 Gew.-% C₈-C₁₂-Alkyloligoglucosid, insbesondere Laurylglucosid,
b) 0,5 -5,0 Gew.-% Polyglycerinpolyhydroxystearat,
c) 0,5 - 5,0 Gew.-% C₁₂-C₁₈-Acylglutamat., insbesondere Kokosfettsäureglutamat,
d) 0,5 - 5,0 Gew.-% Sorbitanester einer C₁₆-C₂₀-Fettsäure, insbesondere Sorbitanstearat,
e) 5,0 - 20,0 Gew.-% Fettsäureester von C₆-C₂₀-Fettsäuren mit C₆-C₂₀-Fettalkoholen, insbesondere Hexyllaurat, Ethylhexylpalmitat und Cetearylisononanoat,
f) 1,0 - 10,0 Gew.-% C₆-C₁₂-Alkylcarbonate, insbesondere Dicaprylylcarbonat, und
g) 0,1 - 5,0 Gew.-% Cyclomethicone und/oder Siliciummethicone, insbesondere Dimethicon,
   sowie
h) 1,0-10 Gew.-% Glycerin,
i) bis zu 90 Gew.-% Wasser und gegebenenfalls
j) aktive Wirkstoffe, Hilfs- und Zusatzstoffe.

Eine weitere bevorzugte Ausführungsform ist dadurch gekennzeichnet, dass die Nanoemulsion im Wesentlichen aus den Komponenten a) - j) besteht. Erfindungsgemäß wird unter "im Wesentlichen" verstanden, dass die Nanoemulsion - bis auf rohstoffbedingten Verunreinigungen - keine anderen als die Komponenten a) - j) umfasst.

### Herstellungsverfahren

Bei der Herstellung der Emulsion kann die erfindungsgemäße Emulgatorkombination insgesamt mit der Ölphase vorgelegt werden. Derzeit bevorzugt ist es jedoch, die Komponenten der Emulgatorkombination auf die Ölphase und die wässrige Phase aufzuteilen und zwar derart, dass die öllöslichen Komponenten der Ölphase zugesetzt werden und die wasserlöslichen der wässrigen Phase.

Im erfindungsgemäßen Verfahren zur Herstellung einer Nanoemulsion wird entsprechend eine den Ölkörper, gegebenenfalls weitere öllösliche Komponenten sowie die Emulgatorkomponenten a) und b) enthaltende Ölphase vorlegt, und in diese wird die wässrige Phase, welche die Emulgatorkomponente c) und gegebenenfalls weitere wasserlösliche Komponenten enthält, unter Rühren einträgt und emulgiert.

Zum Erhalt einer Nanoemulsion ist also ein einfacher Mischvorgang ausreichend. Weder muss ein PIT-Verfahren, eine Hochdruckhomogenisierung noch sonst ein aufwändiges Verfahren durchgeführt werden, um eine stabile Nanoemulsion herzustellen. Die Emulsionen können zudem völlig frei von ethoxylierten Verbindungen hergestellt werden.

Grundsätzlich können mit der erfindungsgemäßen Emulgatorkombination sowohl W/O- als auch O/W-Emulsionen erzeugt werden. Besonders geeignet sind Emulgatorkombination und erfindungsgemäßes Verfahren zur Herstellung phasenstabiler O/W-Emulsionen, die eine niedrige Viskosität (in der Regel unter 100 mPas) aufweisen.

Zweckmäßig geht man beim Eintragen der wässrigen in die Ölphase so vor, dass zunächst etwa 5 bis 10 Vol.-% der wässrigen Phase langsam unter Rühren zugefügt werden. Die Mischung bleibt dabei noch relativ klar. Bei weiterer Zugabe der wässrigen Phase trübt sich die Mischung langsam ein, wird opal und bläulich reflektierend. Schließlich wird eine milchige Emulsion erhalten.

Es wird vermutet, dass der Emulgationsvorgang so abläuft, dass bei Zunahme des Wasseranteils in der Emulsion die Emulgatorkomponenten in der Ölphase zunehmend solubilisiert werden und gleichzeitig das Acylglutamat synergistisch dazu beiträgt, die Hydrophilie der Mischung zu erhöhen, so dass sich auf diese Weise eine O/W-Nanoemulsion bilden kann.

Damit das Acylglutamat seine volle Wirkung entfalten kann und um zu verhindern, dass das Acylglutamat in Form der Glutaminsäure vorliegt, wird zweckmäßig bei einem pH-Wert der wässrigen Phase von mindestens 4,5 gearbeitet. Falls erforderlich, kann die fertige Emulsion anschließend auf den für diese gewünschten pH-Wert gebracht werden.

Über die Einstellung des pH-Wertes in der zu emulgierenden wässrigen Phase kann zudem auch die Teilchen- bzw. Tröpfchengröße in der fertigen Emulsion gesteuert werden. In einer bevorzugten Verfahrensvariante wird entsprechend der pH-Wert der wässrigen Phase in Abhängigkeit von der gewünschten Teilchengröße in der erhaltenen Emulsion eingestellt. Mit steigendem pH-Wert nimmt dabei die Teilchengröße in der Nanoemulsion ab. Dies dürfte auf die stärkere Ionisierung des Acylglutamats zurückzuführen sein. Die zur Einstellung des pH-Wertes verwendbaren Mittel sind dem Fachmann bekannt. Nur beispielhaft seien daher hier Zitronensäure oder Alkalimetallhydroxide genannt.

Das erfindungsgemäße Verfahren ist nicht auf bestimmte Temperaturbereiche beschränkt, sondern kann als Heiß/Heiß- oder als Kalt/Kalt-Verfahren durchgeführt werden. Im Unterschied zum PIT-Verfahren ist die Phaseninversion im erfindungsgemäßen Verfahren nicht temperaturabhängig. Die geeignete Temperatur hängt in an sich bekannter Weise von den verwendeten Komponenten ab. Werden zähflüssige, wachsartige oder feste Komponenten emulgiert, wird man in der Regel bei erhöhten Temperaturen arbeiten. So können beispielsweise Ölphase und wässrige Phase bei einer erhöhten Temperatur von insbesondere 40 bis 80 °C miteinander emulgiert werden.

### Gewerbliche Anwendbarkeit

Die erfindungsgemäße Emulgatorkombination eignet sich zur Herstellung von Naonemulsionen, welche frei von ethoxylierten Emulgatoren sind, die in kosmetischen oder pharmazeutischen Zubereitungen vorteilhaft eingesetzt werden können. Beispiele sind sprühbare Emulsionen für die Körperpflege (Deosprays, Sonnenschutzsprays usw.), Pflegeprodukte in Gel- oder Cremeform, Arzneimittel-haltige Sprays, Gele oder Cremes, getränkte Pflegetücher oder Pads (Make-up-Entferner, Reinigungstücher usw.) und Ähnliches. Die Nanoemulsionen sind besonders geeignet zur Applikation auf Papieren, Tüchern, Textilien und Watteprodukten, die im Bereich der Babypflege und - hygiene sowie im Bereich der Make-up-Entfernung, insbesondere der Augen-Make-up-Entfernung, im Bereich der Damenhygiene (Tampons, Damenbinden, Slip-Einlagen) und im Bereich der Körperhygiene (Toilettenpapier, Feuchttoilettenpapier) eingesetzt werden, da in diesen Bereichen Allergieprobleme besonders häufig auftreten und es gilt, diese Allergieprobleme zu vermeiden.

Marktübliche Reinigungstücher werden entweder mit wäßrigen Lotionen basierend auf ethoxylathaltigen (EO) Emulgatoren, wie z. B. in WO 00/04230 beschrieben oder aber mit wässrigen, klaren Solubilisaten getränkt. Die erstgenannten wässrigen Lotionen werden aufgrund des EO-Gehaltes in verschiedenen Märkten nicht akzeptiert (Öko-Konform). Die klaren Solubilisate sind dagegen häufig sehr klebrig und sensorisch nicht akzeptabel. Da von Solubilisaten aufgrund der Klebrigkeit oft sehr starke Verdünnungen eingesetzt werden müssen, besitzen die damit produzierten Tücher nur noch eine geringe Reinigungsleistung. Die erfindungsgemäßen Nanoemulsionen enthalten geringe Emulgator- und hohe Öl-und Wachsmengen. Daher haben Sie eine sehr hohe Reinigungsleistung. Zudem sind sie frei von ethoxylathaltigen Emulgatoren.

Ein weitere Gegenstand der Anmeldung ist daher die Verwendung der erfindungsgemäßen Nanoemulsion auf Papieren, Vliese (Nonwoven) und Geweben (Woven). Erfindungsgemäß zählen hierzu alle Papierarten, Vliese und Gewebe, die dem Fachmann geläufig sind, und Produkte, die daraus herstellbar sind, wie z.B. Toilettenpapier, Papiertaschentücher, Tissues, Wipes, Watte, Wattepads, Tampons, Binden, Slip-Einlagen, Windeln, Textilien, etc. Ebenso Gegenstand der Anmeldung sind Papier-, Vlies- und Gewebe-Produkte zur Körperpflege und -reinigung, die mit einer erfindungsgemäßen Nanoemulsion beschichet sind.

Besonders vorteilhaft ist es, die Nanoemulsionen mit einem hohen Wassergehalt einzusetzen, damit sie in großtechnischen Verfahren besonders leicht appliziert werden können, z. B. im Sprühverfahren. Derartige Nanoemulsionen haben einen Mindest-Wassergehalt von 60 Gew.-% bezogen auf die Gesamtzusammensetzung der Emulsion, vorzugsweise 70 Gew.-% und insbesondere mehr als 80 Gew.-%. Ein weiterer Gegenstand der Erfindung sind daher Papier-, Vlies- oder Gewebe-Produkte, die mit einer erfindunsggemäßen Nanoemulsion beschichtet sind, die dadurch gekennzeichnet ist, dass sie folgende Komponenten umfasst:
a) 0,10 -3,0 Gew.-% C₈-C₁₂-Alkyloligoglucosid, insbesondere Laurylglucosid,
b) 0,10 -3,0 Gew.-% Polyglycerinpolyhydroxystearat,
c) 0,02 - 3,0 Gew.-% C₁₂-C₁₈-Acylglutamat., insbesondere Kokosfettsäureglutamat,
d) 0,5 - 10,0 Gew.-% einer Ölphase
e) 0,1 - 3 Gew.-% Glycerin,
f) mehr als 60 Gew.-%, vorzugsweise mehr als 80 Gew. % Wasser und gegebenenfalls
g) aktive Wirkstoffe, Hilfs- und Zusatzstoffe.

Die Mengenangaben sind bezogen auf die Gesamtzusammensetzung der Emulsion. Eine weitere bevorzugten Ausführungsform ist dadurch gekennzeichnet, dass die Nanoemulsion im Wesentlichen aus den Komponenten a) - g) besteht. Erfindungsgemäß wird unter "im Wesentlichen" verstanden, dass die Nanoemulsion - bis auf rohstoffbedingten Verunreinigungen - keine anderen als die Komponenten a) - g) umfasst.

Die Papier-, Vlies- oder Gewebe-Produkte können in einem Trocknungsschritt nachbehandelt werden, um den Wassergehalt nach der Sprüh-Applikation zu reduzieren oder um nahezu wasserfreie Produkte zu erhalten (z.B. Dry-Wipes). In einer Ausführungsform der Erfindung werden erfindungsgemäß beschichteten Papier-, Vlies- und Gewebe-Produkte nachfolgend einem Trocknungsschritt unterworfen werden, um das Wasser ganz oder teilweise zu entziehen.

Die Erfindung soll anhand der nachfolgenden Beispiele näher erläutert werden.

### Beispiele

Soweit nicht anders erwähnt, sind die Mengenangaben in den nachfolgenden Beispielen in Gew.-% der handelsüblichen Substanzen oder, wenn keine handelsübliche Subtanz angegeben ist, auf die Menge Aktivsubstanz bezogen auf die Gesamtzusammensetzung (Phase 1 und Phase 2) angegeben. Die Teilchen- bzw. Tröpfchengröße der Emulsionen wurde durch PCS (Photon Correlation Spectroscopy) unter Verwendung eines Spektrometers NICOMP 370 der Pacific Scientific bestimmt.

### Beispiel 1

Es wurden eine Ölphase (Phase 1) und eine wässrige Phase (Phase 2) mit den in Tabelle 1 dargestellten Zusammensetzungen hergestellt. Die Mengenangaben sind mit Bezug auf die fertige Emulsion angegeben, d.h. die Summe der Komponenten der Phasen 1 und 2 ergibt 100 Gew.-%. Beide Phasen wurden auf 70 °C erwärmt. Phase 2 wurde der Ölphase 1 langsam unter Rühren zugesetzt. Dabei blieb die erhaltenen Mischung zunächst bis nach Zugabe von etwa 5 % der wässrigen Phase 2 klar. Bei weiterer Zugabe wurde die Mischung opal, und man konnte eine blaue reflektierende Tönung in der Mischung beobachten. Nach vollständiger Zugabe der Phase 2 und Abkühlen der Mischung auf Raumtemperatur wurde eine milchige phasenstabile Emulsion erhalten.
Die Viskosität der Emulsion lag unter 100 mPas.
Die durchschnittliche Teilchengröße betrug 160 nm.

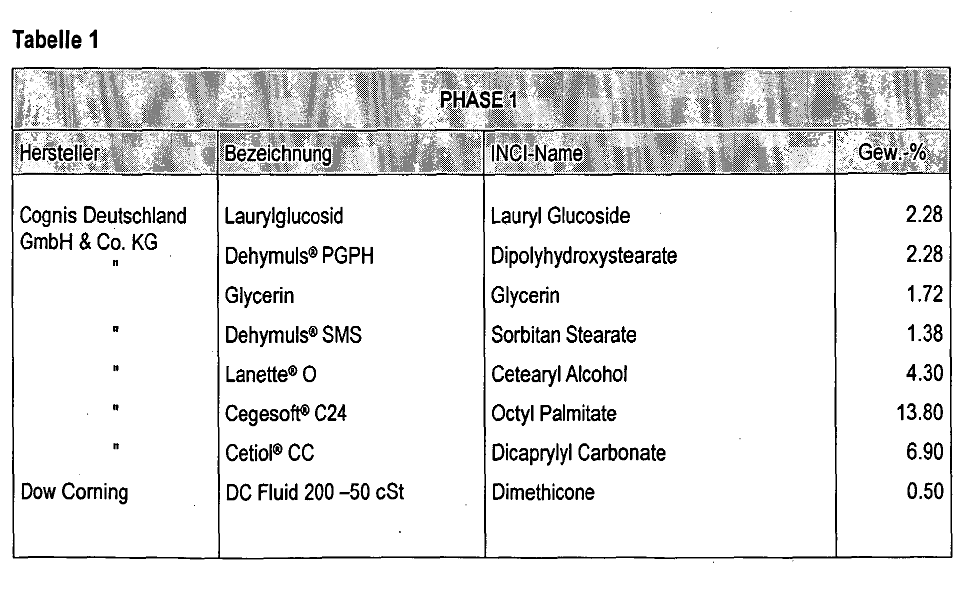

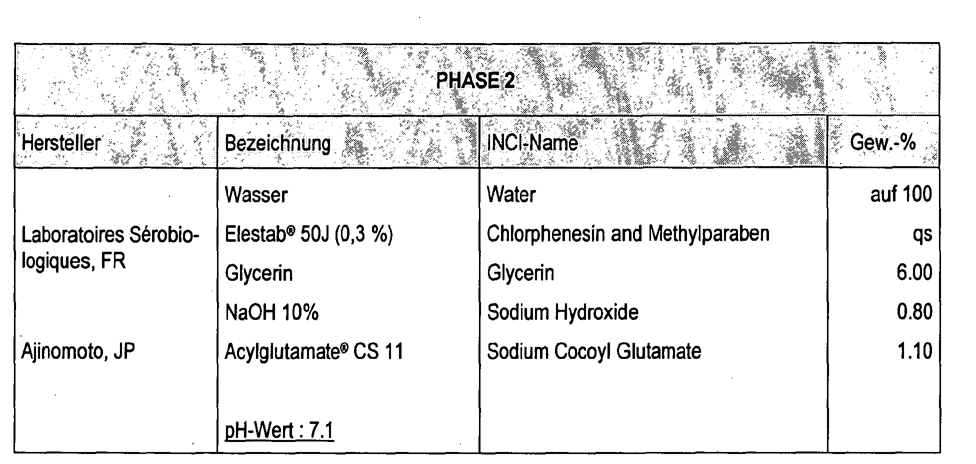

### Beispiel 2

Die Herstellung der Emulsion erfolgte wie in Beispiel 1 unter Verwendung der in Tabelle 2 angegebenen Phasen 1 und 2.
Die Viskosität der Emulsion lag unter 100 mPas.
Die durchschnittliche Teilchengröße betrug 170 nm.

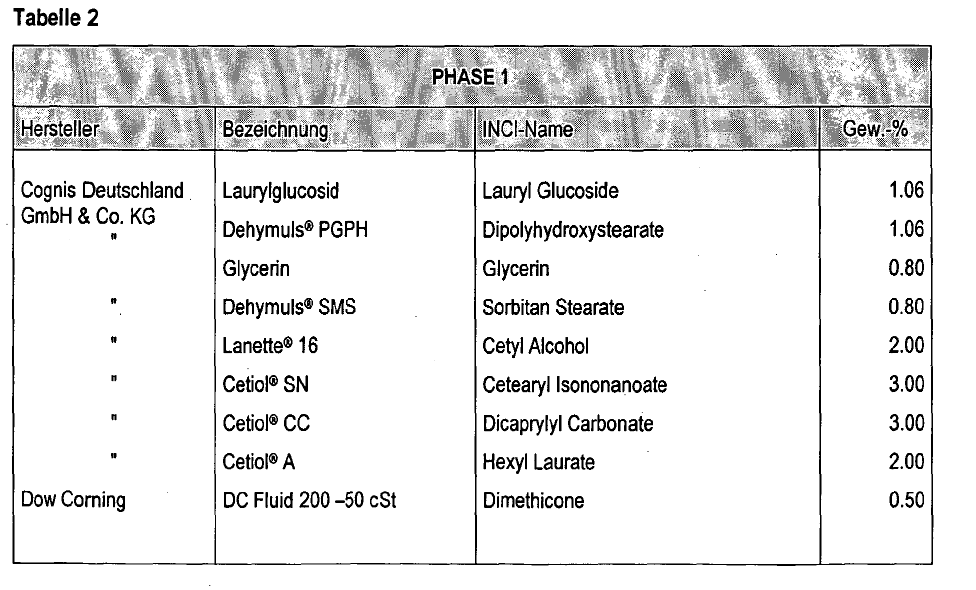

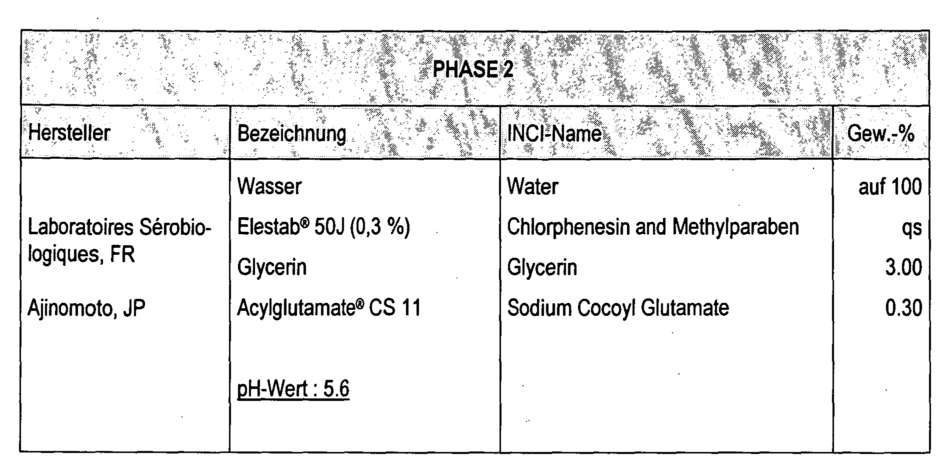

### Beispiel 3

Es wurde die gleiche Emulsion hergestellt wie in Beispiel 2, mit dem einzigen Unterschied, dass der pH-Wert der wässrigen Phase 2 vor dem Mischen mit Phase 1 durch Zugabe von Natriumhydroxid auf 8,5 eingestellt wurde. Die resultierende Nanoemulsion hatte eine Teilchengröße von 110 nm.
Die erhaltene Emulsion wurde anschließend durch Zugabe verdünnter Zitronensäure auf einen pH-Wert von 6.9 eingestellt.

### Beispiel 4

Die in Beispiel 1 erhaltene Nanoemulsion wurde bei Raumtemperatur mit Wasser und einem aktiven Wirkstoff versetzt. Der Wirkstoff Slimfit® LS 9609 der Laboratoires Sérobiologiques, FR dient der kosmetischen, lokalen Gewichtsreduktion.
Durch Mischen von

| | |
|---|---|
| Nanoemulsion nach Beispiel 1 | 50 Gew.-% |
| Wasser | 48 Gew.-% und |
| Slimfit® LS 9609 | 2 Gew.-% |

wurde eine sprühfähige Körperlotion erhalten.

### Beispiel 5

Die Herstellung der Emulsion erfolgte wie in Beispiel 1 unter Verwendung der in Tabelle 3 angegebenen Phasen 1 und 2. Die Emulsion blieb transparent. Die Viskosität der Emulsion lag unter 100 mPa·s.
Die durchschnittliche Teilchengröße betrug 25 nm.

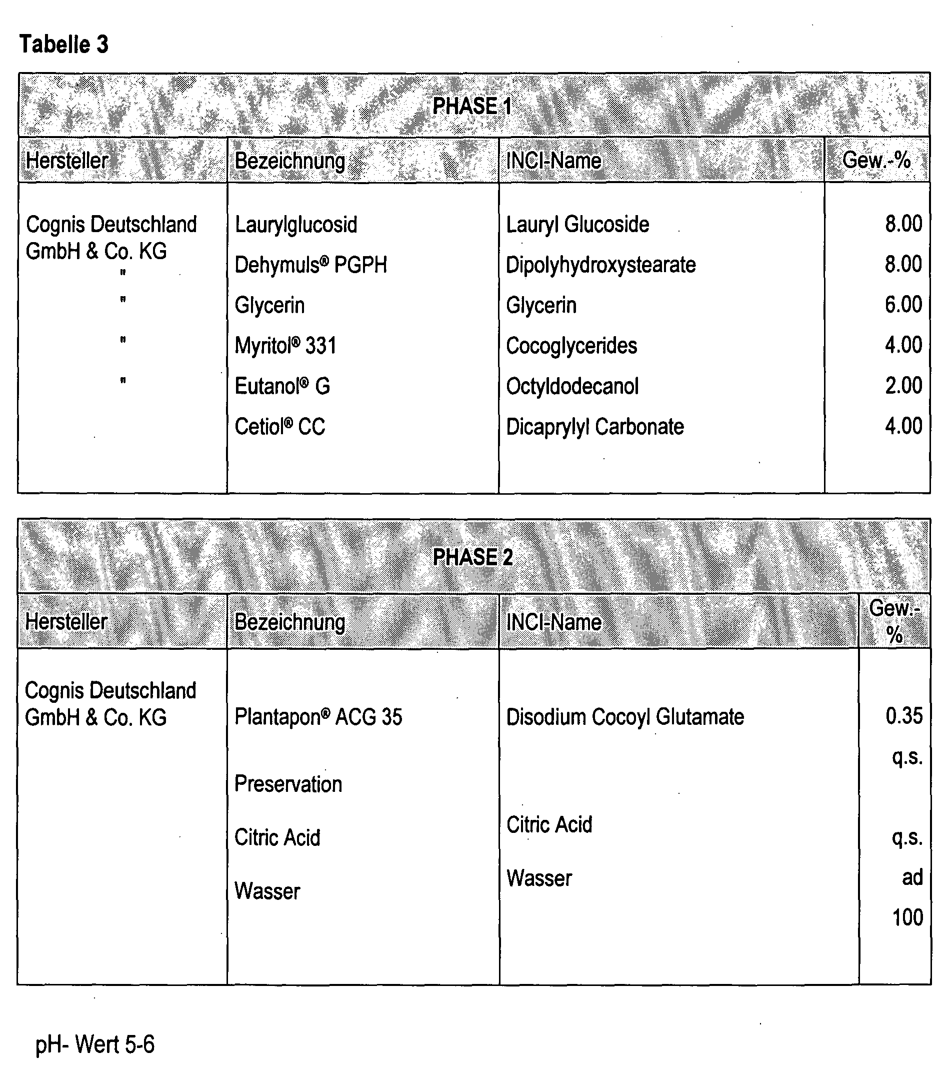

### Beispiel 6

Die Herstellung der Emulsion erfolgte wie in Beispiel 1 unter Verwendung der in Tabelle 4 angegebenen Phasen 1 und 2. Es wurde eine phasenstabile, transparent-bläuliche Emulsion erhalten. Die Viskosität der Emulsion lag unter 100 mPa·s.
Die durchschnittliche Teilchengröße war 32 nm.

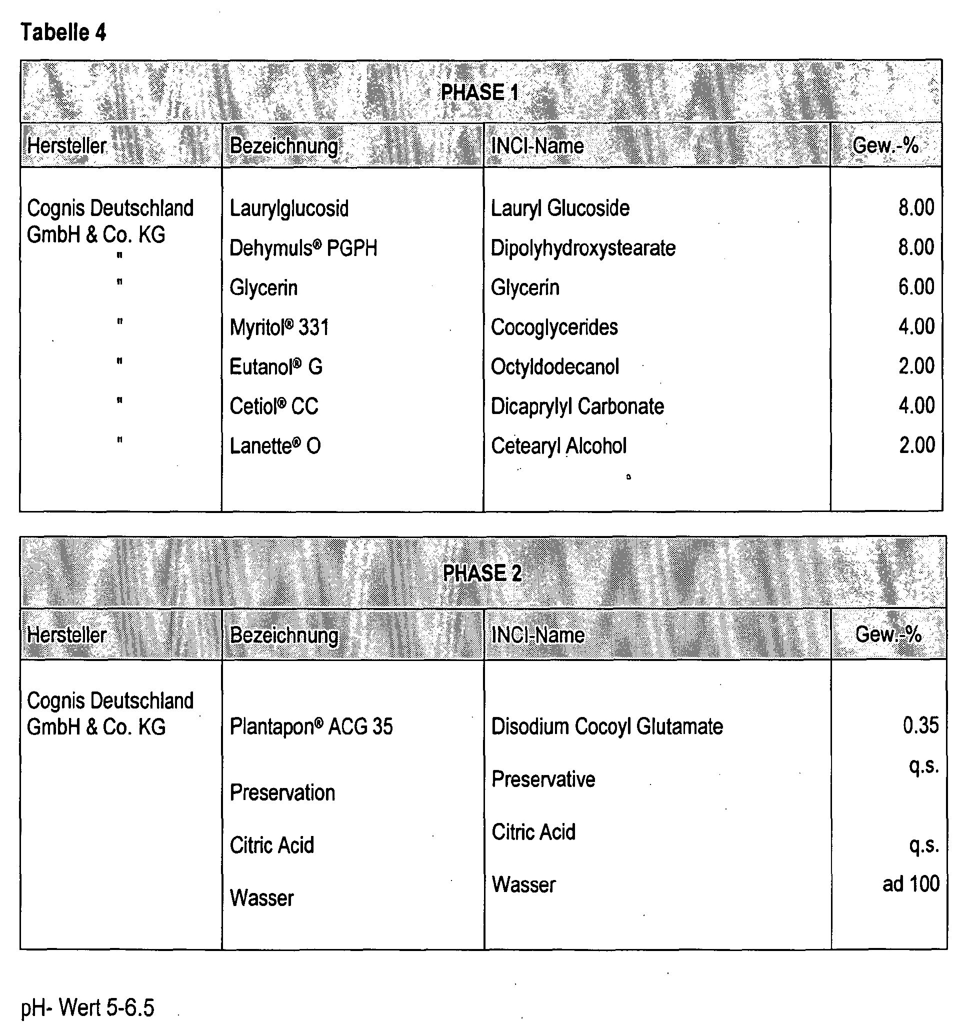

### Beispiel 7

Die Herstellung der Emulsion erfolgte wie in Beispiel 1 unter Verwendung der in Tabelle 5 angegebenen Phasen 1 und 2. Zuerst wurde die Mischung opal, und man konnte eine blaue reflektierende Tönung in der Mischung beobachten. Nach vollständiger Zugabe der Phase 2, Abkühlen der Mischung auf Raumtemperatur und Einstellen des pH's mit Zitronensäure wurde eine milchige phasenstabile Emulsion erhalten. Die Viskosität der Emulsion lag unter 100 mPa·s.
Die durchschnittliche Teilchengröße war 169 nm.

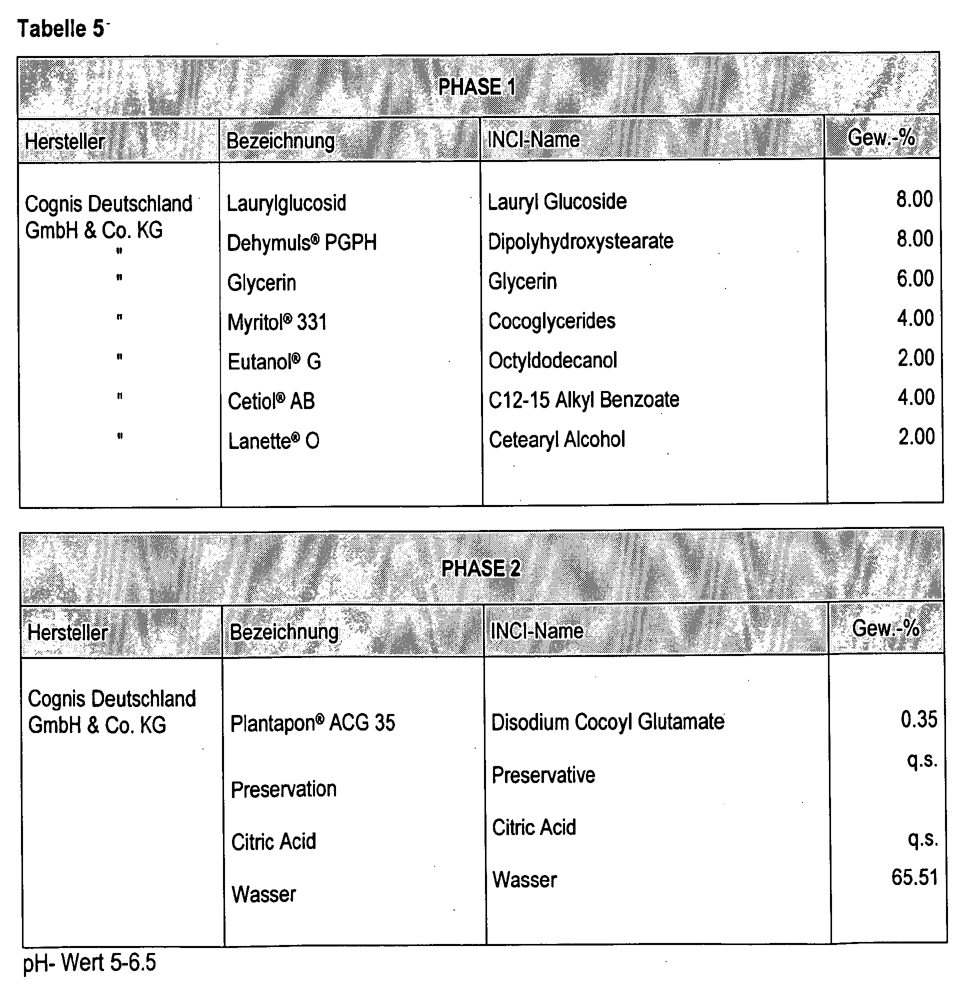

### Beispiel 8:

Die in den Beispiel 1-3 und 5-7 erhaltenen Nanoemulsionen wurden bei Raumtemperatur mit Wasser und Konservierungsstoffen versetzt. Die entstandene Mischung eignet sich besonders als sprühbare Lotion für Reinigungstücher, besonders für das Gesicht und Babyhaut. Durch Mischen von

| | |
|---|---|
| Nanoemulsion nach Beispiel 1-3 und 5-7 | 20 Gew.-% |
| Wasser | 79,0 Gew.-% und |
| Euxyl ® K 702 | 1,0 Gew.-% |

wurde eine sprühfähige Benetzungslösung für Reinigungstücher erhalten.

Die Tücher können besprüht oder aber auch getränkt werden.
Es wurden ca. 3 g der Nanoemulsion pro 1 g des Tuches aufgetragen.
Tuchmaterial: Spunlace Viscose 65% / Polyester 35% - 55 g/m².

## Patentansprüche

1. Emulgatorkombination, welche frei ist von ethoxylierten Emulgatoren, weniger als 20 Gew.-% Wasser enthält und umfasst:
a) C₈-C₁₄-Alkyloligoglykosid
b) Polyolpolyhydroxystearat und
c) C₆-C₂₂-Acylglutamat.

2. Emulgatorkombination gemäß Anspruch 1, worin Komponente a) ein C₈-C₁₂-Alkyloligoglucosid und insbesondere Laurylglucosid ist.

3. Emulgatorkombination gemäß wenigstens einem der Ansprüche 1 bis 2, worin Komponente b) Polyglycerinpolyhydroxystearat ist.

4. Emulgatorkombination gemäß wenigstens einem der Ansprüche 1 bis 3, worin Komponente c) ein C₁₂-C₁₈-Acylglutamat, bevorzugt ein Kokosfettsäureglutamat und insbesondere ein Mono- oder Dialkalimetallsalz des Acylglutamats ist.

5. Emulgatorkombination gemäß wenigstens einem der Ansprüche 1 bis 4, welche mindestens ein weiteres nichtionisches Tensid und insbesondere einen Sorbitanmono- oder -diester einer gesättigten oder ungesättigten Fettsäure mit 6 bis 22 und bevorzugt 12 bis 20 Kohlenstoffatomen, insbesondere Sorbitanstearat, enthält.

6. Emulgatorkombination gemäß wenigstens einem der Ansprüche 1 bis 5, worin der Anteil der Komponente c) bezüglich der Emulgatorkombination a) bis c) 1 bis 50 Gew.-%, bevorzugt 3 bis 15 Gew.-% und insbesondere 5 bis 11 Gew.-% beträgt.

7. Emulgatorkombination gemäß wenigstens einem der Ansprüche 1 bis 6, worin das Gewichtsverhältnis der Komponenten a) und b) 5 : 1 bis 1 : 5 und bevorzugt 2 : 1 bis 1 : 2 beträgt und insbesondere im Wesentlichen gleiche Gewichtsanteile a) und b) enthalten sind.

8. Emulgatorkombination gemäß wenigstens einem der Ansprüche 5 bis 7, worin das Gewichtsverhältnis des weiteren nichtionischen Tensids zur Gesamtmenge der Komponenten a) und b) bei 1 : 1 bis 1 : 10 und bevorzugt bei 1 : 2 bis 1 : 5 liegt.

9. Emulgatorkombination gemäß wenigstens einem der Ansprüche 1 bis 8, in welcher zusätzlich Glycerin enthalten ist.

10. Verwendung der Emulgatorkombination gemäß wenigstens einem der Ansprüche 1 - 9 zur Solubilisierung von Parfümölen.

11. Nanoemulsion, die frei von ethoxylierten Emulgatoren ist, aus einer Ölphase und eine wässrigen Phase, in welcher als Emulgator die Emulgatorkombination nach einem der Ansprüche 1 bis 9 vorhanden ist.

12. Nanoemulsion nach Anspruch 11, in welcher wenigstens ein Ölkörper enthalten ist, der ausgewählt ist aus Fettsäureestern von C₆-C₂₂-Fettsäuren mit C₆-C₂₂-Fettalkoholen, C₆-C₂₂-Dialkylcarbonaten, Siliconölen, 1,3-Dialkylcyclohexanen und C₆-C₂₂-Dialkylethern .

13. Nanoemulsion gemäß wenigstens einem der Ansprüche 11 bis 12, umfassend:
a) C₈-C₁₂-Alkyloligoglucosid, insbesondere Laurylglucosid,
b) Polyglycerinpolyhydroxystearat,
c) C₁₂-C₁₈-Acylglutamat., insbesondere Kokosfettsäureglutamat,
d) Sorbitanester einer C₁₆-C₂₀-Fettsäure, insbesondere Sorbitanstearat,
wenigstens einen Ölkörper und bevorzugt wenigstens einen Ölkörper pro Gruppe, ausgewählt aus:
e) Fettsäureestern von C₆-C₂₀-Fettsäuren mit C₆-C₂₀-Fettalkoholen, insbesondere Hexyllaurat, Ethylhexylpalmitat und Cetearylisononanoat,
f) C₆-C₁₂-Dialkylcarbonaten, insbesondere Dicaprylylcarbonat, und
g) Cyclomethiconen und Siliciummethiconen, insbesondere Dimethicon,
sowie
h) Glycerin,
i) Wasser und gegebenenfalls
j) aktive Wirkstoffe, Hilfs- und Zusatzstoffe.

14. Nanoemulsion gemäß wenigstens einem der Ansprüche 11 bis 13, die zusätzlich C₁₆-C₂₂-Fettalkohol(e) enthält, insbesondere in einer Menge von 1,0 -10 Gew.-% und insbesondere Cetyl- oder Cetylstearylalkohol.

15. Nanoemulsion gemäß wenigstens einem der Ansprüche 11 bis 14, worin der Anteil an Komponente c) bei 0,05 bis 3 Gew.-% und insbesondere bei 0,1 bis 1,5 Gew.-%, bezogen auf die Gesamtmenge der Nanoemulsion, liegt.

16. Nanoemulsion gemäß wenigstens einem der Ansprüche 11 bis 15, die weitere Emulgatorkomponenten in einer Menge von 0,1 bis 15 Gew.-% und insbesondere 1 bis 10 Gew.-%, bezogen auf die Gesamtmenge der Nanoemulsion, enthält.

17. Nanoemulsion gemäß wenigstens einem der Ansprüche 11 bis 16, worin der Anteil an Ölkörper 1 bis 50 Gew.-% und insbesondere 1 bis 30 Gew.-%, bezogen auf die Gesamtmenge der Nanoemulsion, beträgt.

18. Nanoemulsion gemäß Anspruch 13, umfassend:
a) 0,5 -5,0 Gew.-% C₈-C₁₂-Alkyloligoglucosid, insbesondere Laurylglucosid,
b) 0,5 -5,0 Gew.-% Polyglycerinpolyhydroxystearat,
c) 0,5 - 5,0 Gew.-% C₁₂-C₁₈-Acylglutamat., insbesondere Kokosfettsäureglutamat,
d) 0,5 - 5,0 Gew.-% Sorbitanester einer C₁₆-C₂₀-Fettsäure, insbesondere Sorbitanstearat,
e) 5,0 - 20,0 Gew.-% Fettsäureester von C₆-C₂₀-Fettsäuren mit C₆-C₂₀-Fettalkoholen, insbesondere Hexyllaurat, Ethylhexylpalmitat und Cetearylisononanoat,
f) 1,0 - 10,0 Gew.-% C₆-C₁₂-Alkylcarbonate, insbesondere Dicaprylylcarbonat, und
g) 0,1 - 5,0 Gew.-% Cyclomethicone und/oder Siliciummethicone, insbesondere Dimethicon,
sowie
h) 1,0 -10 Gew.-% Glycerin,
i) bis zu 90 Gew.-% Wasser und gegebenenfalls
j) aktive Wirkstoffe, Hilfs- und Zusatzstoffe.

19. Verfahren zur Herstellung einer Nanoemulsion gemäß wenigstens einem der Ansprüche 11 bis 18, in welchem man eine den Ölkörper, gegebenenfalls weitere öllösliche Komponenten sowie die Emulgatorkomponenten a) und b) enthaltende Ölphase vorlegt und in diese die wässrige Phase, welche die Emulgatorkomponente c) und gegebenenfalls weitere wasserlösliche Komponenten enthält, unter Rühren einträgt und emulgiert.

20. Verfahren nach Anspruch 19, worin man Ölphase und wässrige Phase bei erhöhter Temperatur, insbesondere bei 40 bis 80 °C, miteinander emulgiert.

21. Verfahren gemäß wenigstens einem der Ansprüche 19 bis 20, worin die wässrige Phase einen pH-Wert von mindestens 4,5 aufweist.

22. Verfahren nach Anspruch 21, worin der pH-Wert der wässrigen Phase in Abhängigkeit von der gewünschten Teilchengröße in der erhaltenen Emulsion eingestellt und insbesondere zur Erzielung geringerer Teilchengrößen erhöht wird.

23. Verwendung einer Nanoemulsion gemäß wenigstens einem der Ansprüche 11 - 18 auf Papieren, Vliesen und Geweben.

24. Papier-, Vlies- und Gewebe-Produkte zur Körperpflege und -reinigung, die mit einer Nanoemulsion gemäß wenigstens einem der Ansprüche 11 - 18 beschichet ist.

25. Papier-, Vlies- und Gewebe-Produkte, die mit einer Nanoemulsion gemäß Anspruch 11 beschichtet ist, **dadurch gekennzeichnet, dass** die Nanoemulsion folgende Komponenten umfasst:
a) 0,1 -3,0 Gew.-% C₈-C₁₂-Alkyloligoglucosid, insbesondere Laurylglucosid,
b) 0,1 -3,0 Gew.-% Polyglycerinpolyhydroxystearat,
c) 0,02 - 3,0 Gew.-% C₁₂-C₁₈-Acylglutamat., insbesondere Kokosfettsäureglutamat,
d) 0,5 - 10,0 Gew.-% einer Ölphase
e) 0,1 - 3 Gew.-% Glycerin,
f) mehr als 60 Gew.-%, vorzugsweise mehr als 80 Gew. % Wasser und gegebenenfalls
g) aktive Wirkstoffe, Hilfs- und Zusatzstoffe.

26. Papier-, Vlies- und Gewebe-Produkte gemäß Anspruch 24 oder 25, die nachfolgend einem Trocknungsschritt unterworfen werden, um das Wasser ganz oder teilweise zu entziehen.
